(19) Europäisches Patentamt
European Patent Office
Office européen des brevets

(11) Publication number: **0 444 961 A1**

# EUROPEAN PATENT APPLICATION

(21) Application number: **91301737.2**

(22) Date of filing: **01.03.91**

(51) Int. Cl.⁵: **C12N 15/12, C07H 21/04,** **C12P 21/00**

(30) Priority: **02.03.90 US 488347**

(43) Date of publication of application:
**04.09.91 Bulletin 91/36**

(84) Designated Contracting States:
**AT BE CH DE DK ES FR GB GR IT LI LU NL SE**

(71) Applicant: **Bristol-Myers Squibb Company**
**345 Park Avenue**
**New York, N.Y. 10154 (US)**

(72) Inventor: **Plowman, Gregory D.**
**1000 Union Street No. C.**
**Seattle, Washington 98101 (US)**
Inventor: **Shoyab, Mohammed**
**2405 Westmont Way West**
**Seattle, Washington 98199 (US)**
Inventor: **Whitney, Gena S.**
**2150 6th Avenue North, no.102**
**Seattle, Washington 98109 (US)**

(74) Representative: **Jones, Alan John et al**
**CARPMAELS & RANSFORD 43 Bloomsbury**
**Square**
**London, WC1A 2RA (GB)**

(54) Her3: A novel EGF receptor homolog.

(57)   The cloning, sequence and expression of a novel member of the EGFR/*neu* subfamily of receptor tyrosine kinases, termed HER3, is described. The HER3 precursor comprises 1342 amino acid residues, 1323 residues of which comprise the mature HER3. Recombinant HER3 does not interact with the homologous ligands EGF and TGF-α. A 160 kD phosphoprotein is present in cells expressing both HER3 and membrane-associated Amphiregulin, whereas the 170 kD EGF receptor is completely absent. Immunoprecipitation experiments suggest this 160 kD phosphoprotein is HER3 and that it is present only when coexpressed with the integral membrane form of the AR precursor.

EP 0 444 961 A1

Fig. 1

1 CTCTCACACACACACACCCCTCCCCTGCCATCCCTCCCGGGACTCCGGCTCCGGCTCCGATTGCAATTTGCAACCTCCGCTGCCGTCGCCGCAGCAGCCACCAATTCGCCAGCGGTTCAG

...

# HER3: A NOVEL EGF RECEPTOR HOMOLOG

## 1. INTRODUCTION

The present invention is directed to HER3, a novel receptor tyrosine kinase which is related to the Epidermal Growth Factor Receptor (EGFR) and HER2/*neu*, to nucleotide sequences encoding HER3, and to methods for the generation of DNAs encoding HER3 and their gene products. The HER3 DNAs and polypeptides of the invention may be useful in the diagnosis and treatment of certain abnormalities involving HER3 and/or EGFR and HER2/*neu*, such as certain neoplastic diseases.

## 2. BACKGROUND OF THE INVENTION

Cell growth and differentiation are regulated in part by the specific interaction of secreted growth factors and their membrane-bound receptors. Receptor-ligand interaction results in activation of intercellular signals leading to specific cellular responses. EGF, platelet-derived growth factor (PDGF), insulin, insulin-like growth factor-1, colony stimulating factor-1, and fibroblast growth factor all transmit their growth modulating signals by binding to and activating receptors with intrinsic tyrosine kinase activity (reviewed in Hanks et al., 1988, Science 241: 42-52). Characterization of the physiologic and chemical effects that result from the binding of EGF and TGF-$\alpha$ to the EGF receptor has served as a useful model for understanding receptor-ligand interactions, signal transduction, and the regulation of cell growth and oncogenesis (reviewed in Yarden and Ullrich, 1988, Biochemistry 27: 3113-19)

The EGF receptor (EGFR) is a 170 kD transmembrane protein possessing intrinsic tyrosine kinase activity (reviewed in Hunter and Cooper, 1985, Ann. Rev. Biochem. 54: 897-930). HER2/*neu* is a 185 KD transmembrane receptor protein and the product of the neu proto-oncogene; HER2/*neu* is structurally similar to EGFR and, like EGFR, HER2/*neu* also possesses tyrosine kinase activity (Stern et al., 1986, Mol. Cell. Biol. 6: 1729-40; Akiyama et al., 1986, Science 232: 1644-46). The ligand for HER2/*neu* is not known. Reports from several groups indicate that both EGFR and HER2/*neu* are involved with the development and/or maintenance of human cancers. In this regard, overexpression of EGFR in squamous cell carcinomas and glioblastomas, and overexpression of HER2/*neu* in breast and ovarian carcinomas, have been reported.

## 2.1. AMPHIREGULIN

Amphiregulin (AR) was originally identified from phorbol ester treated human breast carcinoma cells, based on its ability to inhibit the growth of several carcinoma cell lines while stimulating proliferation of normal cells The cloning and expression of Amphiregulin is described in co-pending GB-A-2 214 185, which application is incorporated by reference herein in its entirety. AR shares 38% sequence identity with Epidermal Growth Factor (EGF), and conserves most of the residues proposed to be involved in binding to the EGF receptor (EGFR). However, mature AR contains a distinct amino-terminal sequence of 43 predominantly hydrophilic amino acids, not present in other EGF-like proteins.

## 3. SUMMARY OF THE INVENTION

The present invention is directed to HER3, a novel receptor tyrosine kinase related to the EGF receptor and HER2/*neu*, nucleic acid molecules and vectors encoding HER3, methods for the recombinant synthesis of HER3, anti-HER3 antibodies, and diagnostic and therapeutic uses thereof.

In specific embodiments, described by way of the examples in Sections 6 and 7, *infra*, the cloning and expression of HER3/c-erbB-3 is described. The 6 kb HER3/c-erbB-3 transcript has been identified in various human tissues. HER3, while structurally similar to EGFR and HER2/*neu*, does not bind EGF or TGF-$\alpha$. However, HER3 is specifically phosphorylated when co-expressed with the Amphiregulin precursor.

## 4. BRIEF DESCRIPTION OF THE FIGURES

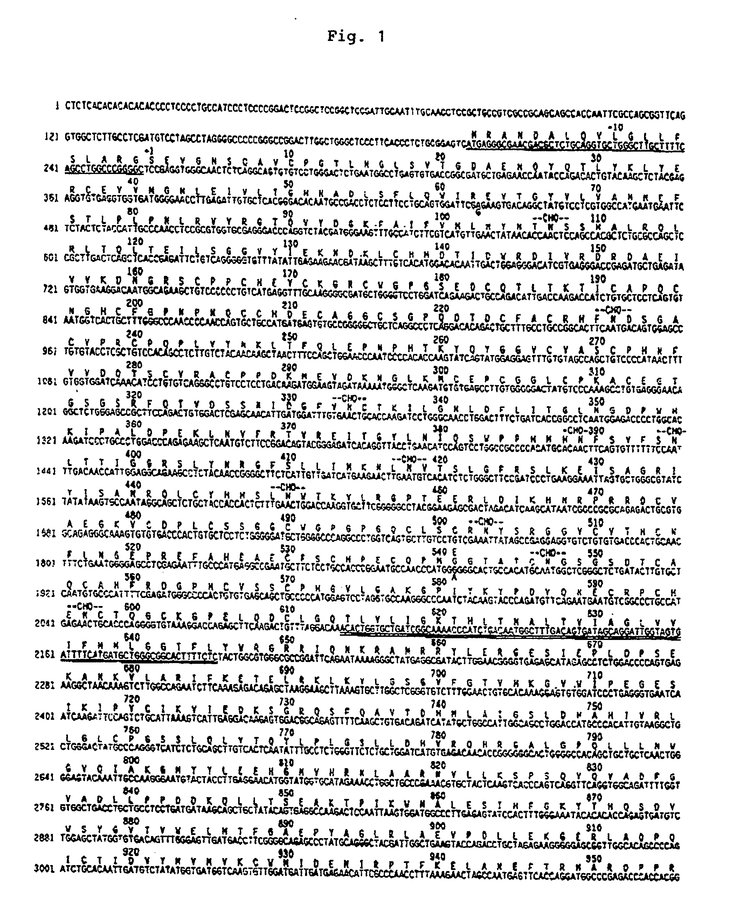

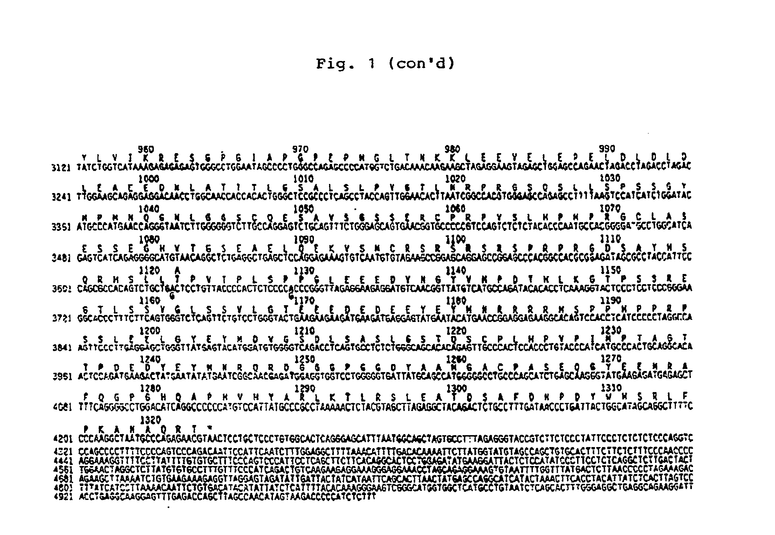

FIG. 1 Nucleotide sequence and deduced amino acid sequence of HER3 (1342 residues). Nucleotides are numbered on the left, and amino acids are numbered above the sequence. The 19 residue signal sequence is underscored with a single line, and the predicted amino-terminus of the mature protein is indicated by +1. The transmembrane domain is marked with a double underline at amino acids 614 - 645. Potential N-linked glycosylation sites (NXS, or NXT) are denoted with -CHO-. No poly(A) tail was present in the HER3 clones. This sequence represents a consensus based on cDNA and PCR clones isolated from MDA-MB-361 RNA, and was used in subsequent expression constructs. Clonal sequence differences are listed below the consensus residue, and include: $A^{441}$ to G in one of 4 PCR clones; $G^{1877}$ to A from A431 cDNA clone; $A^{3619}$ to G in one cDNA clone; $A^{3642}$ to G in one cDNA clone.

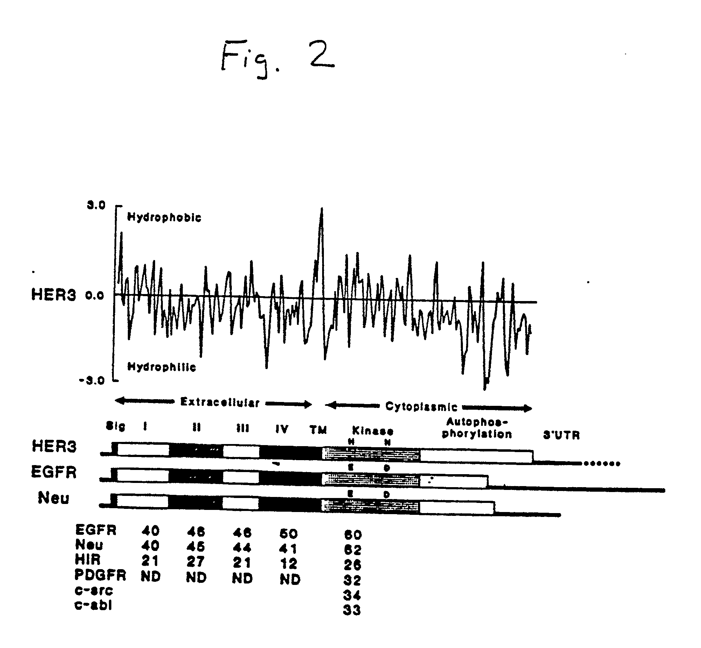

FIG. 2 Hydropathy profile of HER3 and comparison of protein domains for HER3 (1342 amino acids), EGFR (1210 amino acids), and neu/c-erbB-2 (1255 amino acids). The signal peptide and transmembrane domains are represented by filled boxes, the cysteine-rich extracellular subdomains are hatched, and the cytoplasmic tyrosine kinase domain is stippled. The percent amino acid sequence identity between HER3 and representatives of other classes of tyrosine kinases are indicated. Sig, signal peptide; I, II, III, and IV, extracellular domains; TM, transmembrane domain; 3'UTR, 3' untranslated region; HIR, insulin receptor; PDGFR, platelet-derived growth factor receptor; ND, not determined.

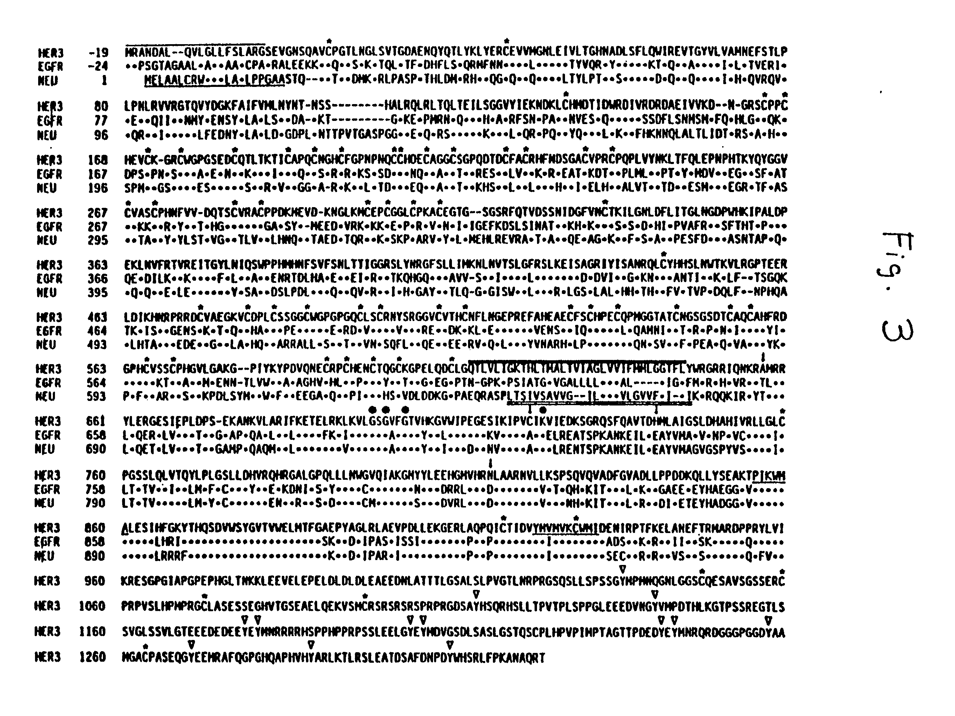

FIG. 3 Protein sequence comparison between members of the human EGF receptor family. Sequences are displayed using the single-letter code, with identical residues denoted with dots. Gaps are introduced for optimal alignment and are show by a dash. The signal sequences are bounded by single lines, the transmembrane domains are bounded by double lines, and sequences used to derive the probes and primers (ARRD1, ARRD2) used for cloning are underlined. Cysteine residues are marked with asterisks, the potential ATP-binding site is shown with circled crosses (G-X-G-X-X-G beginning at residue 697, and K at 723), carboxyl-terminal tyrosines are denoted with open triangles, and additional residues of HER3 that are referred to in Section 6., infra are marked with arrows ($A^{657}$, $C^{721}$, $H^{740}$ and $N^{816}$).

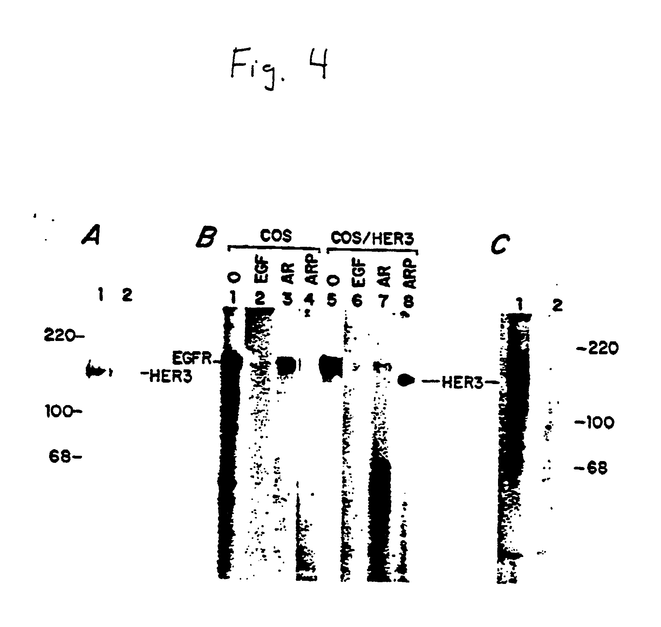

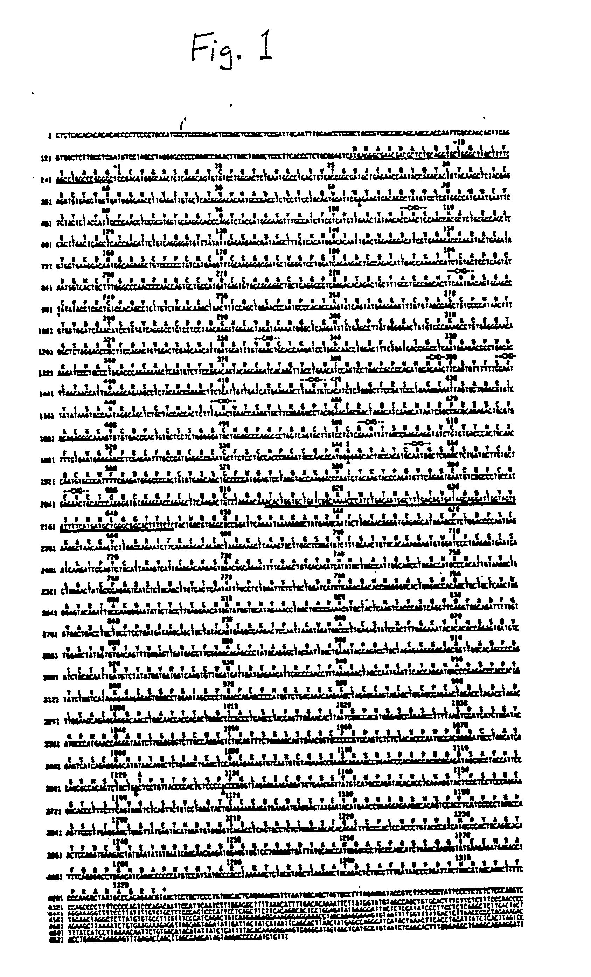

FIG. 4 Differential effects of AR on EGFR and HER3. (A) Immunoblot analysis of the recombinant HER3 transiently expressed in COS cells. The complete HER3 coding sequence was reconstructed in a cDM8 expression vector as described in Section 7.1.1, infra. Three days after transfection, cells were lysed in SDS-sample buffer, protein (100 μg per lane) was resolved on 7% SDS gels, and immunoblotted using HER3-specific antisera. Lane 1, HER3 transfected COS cells; lane 2, cDM8 mock transfected COS cells. Note the presence of a 160 kD band. (B) Amphiregulin blocks phosphorylation of EGFR and stimulates phosphorylation of HER3. Immune complex kinase assays were performed on COS cells transfected with cDM8 vector alone (lanes 1-3), pH3ARP (lane 4), cHER3x (lanes 5-7), or cHER3x and pH3ARP (lane 8), and treated with EGF (lanes 2, 6), AR (lanes 3, 7), or buffer control (lanes 1, 4, 5, 8) as described in Section 7.1.2. infra. (C) Specific immunoprecipitation of phosphorylated HER3 from in vivo labeled COS cells transfected with cHER3x and pH3ARP (lane 1) compared to mock transfected controls (lane 2).

## 5. DETAILED DESCRIPTION OF THE INVENTION

The present invention is directed to what applicants have termed "HER3", a closely related yet distinct member of the Human EGF Receptor (HER)/neu subfamily of receptor tyrosine kinases, HER3-encoding nucleic acid molecules (e.g., cDNAs, genomic DNAs, RNAs, anti-sense RNAs, etc.), production of mature and precursor HER3 from a HER3 gene sequence and its product, recombinant HER3 expression vectors, HER3 analogues and derivatives, HER3 antibodies, and diagnostic and/or therapeutic uses of HER3 and related proteins, HER3-encoding nucleic acid molecules, anti-HER3 antibodies, HER3 ligands, and HER3 antagonists.

### 5. 1. PRODUCTION OF RECOMBINANT HER3

Mature biologically active HER3 may be produced by the cloning and expression of the HER3 precursor coding sequence or its functional equivalent in a host cell which processes the precursor correctly. Functional equivalents of the HER3 precursor coding sequence include any DNA sequence which, when expressed inside the appropriate host cell, is capable of directing the synthesis, processing and/or export of mature HER3.

Production of an HER3 using recombinant DNA technology may be divided into a four-step process for the purposes of description: (1) isolation or generation of the coding sequence (gene) for a precursor or mature form of the desired HER3; (2) construction of an expression vector capable of directing the synthesis of the desired HER3; (3) transfection or transformation of appropriate host cells capable of replicating and expressing the HER3 gene and/or processing the gene product to produce the desired HER3; and (4) identification and purification of the desired HER3 product.

### 5.1.1. ISOLATION OR GENERATION OF THE HER3 GENE

The nucleotide coding sequences of HER3, or functional equivalents thereof, may be used to construct recombinant expression vectors which will direct the expression of the desired HER3 product. The nucleotide coding sequence for HER3 is depicted in FIG. 1. In the practice of the method of the invention, the nucleotide sequence depicted therein, or fragments or functional equivalents thereof, may be used to generate the recombinant molecules which will direct the expression of the recombinant HER3 product in appropriate host cells. HER3-encoding nucleotide sequences may be obtained from a variety of cell sources which produce HER3-like activities and/or which express HER3-encoding mRNA. Applicants have identified a number of suitable human cell sources for HER3 including epidermoid and breast carcinoma cells, normal colon, kidney, and brain.

The HER3 coding sequence may be obtained by cDNA cloning from RNA isolated and purified from such cell sources or by genomic cloning. Either cDNA or genomic libraries of clones may be prepared using techniques well known in the art and may be screened for particular HER3-DNAs with nucleotide probes which are substantially complementary to any portion of the HER3 gene. Full length clones, i.e., those containing the entire coding region of the desired HER3 may be selected for constructing expression vectors. Alternatively, HER3-encoding DNAs may be synthesized in whole or in part by chemical synthesis using techniques standard in the art.

Due to the inherent degeneracy of nucleotide coding sequences, other DNA sequences which encode substantially the same or a functionally equivalent amino acid sequence may be used in the practice of the method of the invention. Such alterations of HER3 nucleotide sequences include deletions, additions or substitutions of different nucleotides resulting in a sequence that encodes the same or a functionally equivalent gene product. The gene product may contain deletions, additions or substitutions of amino acid residues within the sequence which result in silent changes thus producing a bioactive product. Such amino acid substitutions may be made on the basis of similarity in polarity, charge, solubility, hydrophobicity, hydrophilicity and/or the amphipathic nature of the resides involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine; glycine, alanine; asparagine, glutamine; serine, threonine; phenylalanine, tyrosine.

## 5.1.2. CONSTRUCTION OF HER3 EXPRESSION VECTORS

In order to express biologically active, precursor or mature forms of HER3, an expression vector/host system should be chosen which provides not only for high levels of transcription and translation but for the correct processing and post-translational modification of the gene product. This may be especially important when employing the entire coding sequence of the HER3 precursor in the expression constructs since the mature form of HER3 appears to be derived from a larger precursor via cellular processing events.

A variety of animal/host expression vector systems (i.e., vectors which contain the necessary elements for directing the replication, transcription and translation of the HER3 coding sequence in an appropriate host cell) may be utilized equally well by the skilled artisan. These include, but are not limited to, virus expression vector/mammalian host cell systems (e.g., cytomegalovirus, vaccinia virus, adenovirus, and the like); insect virus expression vector/insect cell systems (e.g., baculovirus); or nonviral promoter expression systems derived from the genomes of mammalian cells (e.g., the mouse metallothionein promoter).

The expression elements of these vectors vary in their strength and specificities. Depending on the host/vector system utilized, any one of a number of suitable transcription and translation elements may be used. For instance, when cloning in mammalian cell systems, promoters isolated from the genome of mammalian cells, (e.g. mouse metallothionein promoter) or from viruses that grow in these cells, (e.g. vaccinia virus 7.5K promoter or Moloney murine sarcoma virus long terminal repeat) may be used. Promoters produced by recombinant DNA or synthetic techniques may also be used to provide for transcription of the inserted sequences.

Specific initiation signals are also required for sufficient translation of inserted protein coding sequences. These signals include the ATG initiation codon and adjacent sequences. In cases where an entire HER3 gene including its own initiation codon and adjacent sequences are inserted into the appropriate expression vectors, no additional translational control signals may be needed. However, in cases where only a portion of the coding sequence is inserted, exogenous translational control signals, including the ATG initiation codon must be provided. Furthermore, the initiation codon must be in phase with the reading frame of the HER3 coding sequences to ensure translation of the entire insert. These exogenous translational control signals and initiation codons can be of a variety of origins, both natural and synthetic. The efficiency of expression may be enhanced by the inclusion of transcription attenuation sequences, enhancer elements, etc.

Any of the methods previously described for the insertion of DNA fragments into a vector may be used to construct expression vectors containing the HER3 gene and appropriate transcriptional/translational control signals. These methods may include in vitro recombinant DNA techniques, synthetic techniques and in vivo recombinations.

For example, in cases where an adenovirus is used as an expression vector, the HER3 coding sequence may be ligated to an adenoviris transcription/translation control complex, e.g., the late promoter and tripartite leader sequence. This chimeric gene may then be inserted in the adenovirus genome by in vitro or in vivo recombination. Insertion may be into a non-essential region of the viral genome that is viable and capable of expressing the HER3 in infected hosts. Similarly, the vaccinia 7.5K promoter may be used.

An alternative expression system which could be used to express HER3 is an insect system. In one such system, Autographa californica nuclear polyhedrosis virus (AcNPV) is used as a vector to express foreign genes. The virus grows in Spodoptera frugiperda cells. The HER3 coding sequence may be cloned into nonessential regions (for example the polyhedrin gene) of the virus and placed under control of an AcNPV promoter (for example the polyhedrin promoter). Successful insertion of the HER3 coding sequence will result in inactivation of the polyhedrin gene and production of non-occluded recombinant virus (i.e., virus lacking the proteinaceous coat codes for by the polyhedrin gene). These recombinant viruses are then used to infect Spodoptera frugiperda cells in which the inserted gene is expressed.

Retroviral vectors prepared in amphotropic packaging cell lines permit high efficiency expression in numerous cell types. This method allows one to assess cell-type specific processing, regulation or function of the inserted protein coding sequence.

In addition, a host cell strain may be chosen which modulates the expression of the inserted sequences, or modifies and processes the gene product in the fashion desired. Expression from certain promoters can be elevated in the presence of certain inducers, (e.g. zinc and cadmium ions for metallothionein promoters). Therefore, expression of HER3 may be controlled. This is important if the protein product of the cloned foreign gene is lethal to host cells. Furthermore, modifications (e.g. glycosylation) and processing (e.g., cleavage) of protein products are important for the function of the protein. Different host cells have characteristic and specific mechanisms for the post-translational processing and modification of proteins. Appropriate cell lines or host systems can be chosen to ensure the correct modification and processing of the foreign protein expressed.

### 5.1.3. IDENTIFICATION OF TRANSFECTANTS OR TRANSFORMANTS EXPRESSING HER3 GENE PRODUCTS

The host cells which contain the recombinant coding sequence and which express the biologically active, mature product may be identified by at least four general approaches (a) DNA-DNA, DNA-RNA or RNA-antisense RNA hybridization; (b) the presence or absence of "marker" gene functions; (c) assessing the level of transcription as measured by the expression of HER3 mRNA transcripts in the host cell; and (d) detection of the mature gene product as measured by immunoassay and, ultimately, by its biological activities.

In the first approach, the presence of HER3 coding sequences inserted into expression vectors can be detected by DNA-DNA hybridization using probes comprising nucleotide sequences that are homologous to the HER3 coding sequence.

In the second approach, the recombinant expression vector/host system can be identified and selected based upon the presence or absence of certain "marker" gene functions (e.g., thymidine kinase activity, resistance to antibiotics, resistance to methotrexate, transformation phenotype, occlusion body formation in baculovirus, etc.). For example, if the HER3 coding sequence is inserted within a marker gene sequence of the vector, recombinants containing that coding sequence can be identified by the absence of the marker gene function. Alternatively, a marker gene can be placed in tandem with the HER3 sequence under the control of the same or different promoter used to control the expression of the HER3 coding sequence. Expression of the marker in response to induction or selection indicates expression of the HER3 coding sequence.

In the third approach, transcriptional activity for the HER3 coding region can be assessed by hybridization assays. For example, polyadenylated RNA can be isolated and analyzed by Northern blot using a probe homologous to the HER3 coding sequence or particular portions thereof. Alternatively, total nucleic acids of the host cell may be extracted and assayed for hybridization to such probes.

In the fourth approach, the expression of HER3 can be assessed immunologically, for example by Western blots, immunoassays such as radioimmunoprecipitation, enzyme-linked immunoassays and the like. The ultimate test of the success of the expression system, however, involves the detection of the biologically active HER3 gene product. Where the host cell secretes the gene product the cell free media obtained from the cultured transfectant host cell may be assayed for HER3 activity. Where the gene product is not secreted, cell lysates may be assayed for such activity. In either case, assays which measure ligand binding to HER3 or other bioaetivities of HER3 may be used.

### 5. 2. HER3 DERIVATIVES, ANALOGS AND PEPTIDES

The production and use of derivatives, analogues, and peptides related to HER3 are also envisioned and are within the scope of the invention. Such derivatives, analogues, or peptides may have enhanced or diminished biological activities in comparison to native HER3. HER3-related derivatives, analogues, and peptides of the invention may be produced by a variety of means known in the art. Procedures and manipulations at the genetic and protein levels are within the scope of the invention. At the protein level, numerous chemical modifications could be used to produce HER3-like derivatives, analogues, or peptides by techniques known in the art, including but not limited to specific chemical cleavage by endopeptidases (e.g. cyanogen bromides, trypsin, chymotrypsin, V8 protease, and the like) or exopeptidases, acetylation, formylation, oxidation, etc.

## 5.3. ANTI-HER3 ANTIBODIES

Also within the scope of the invention is the production of polyclonal and monoclonal antibodies which recognize HER3 or related proteins.

Various procedures known in the art may be used for the production of polyclonal antibodies to epitopes of HER3. For the production of antibodies, various host animals can be immunized by injection with HER3, or a synthetic HER3 peptide, including but not limited to rabbits, mice, rats, etc. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, oil emulsions, keyhole lympet hemocyanins, dinitrophenol, and potentially useful human adjuvants such as BCG (bacille Calmette-Guerin) and Corynebacterium parvum.

A monoclonal antibody to an epitope of HER3 can be prepared by using any technique which provides for the production of antibody molecules by continuous cell lines in culture. These include but are not limited to the hybridoma technique originally described by Kohler and Milstein (1975, Nature 256, 495-497), and the more recent human B-cell hybridoma technique (Kosbor et al., 1983, Immunology Today 4:72) and EBV-hybridoma technique (Cole et al., 1985, Monoclonal Antibodies and Cancer Therapy, Alan R. Liss, Inc., pp. 77-96).

Antibody fragments which contain the idiotype of the molecule can be generated by known techniques. For example, such fragments include but are not limited to: the $F(ab')_2$ fragment which can be produced by pepsin digestion of the antibody molecule; the Fab' fragments which can be generated by reducing the disulfide bridges of the $F(ab')_2$ fragment, and the two Fab fragments which can be generated by treating the antibody molecule with papain and a reducing agent.

Antibodies to HER3 may find use in the qualitative detection of mature HER3 and their precursor and subcomponent forms, in the affinity purification of HER3 polypeptides, and in the elucidation of HER3 biosynthesis, metabolism and function. Antibodies to HER3 may also be useful as diagnostic and therapeutic agents.

## 5.4. USES OF HER3, HER3-ENCODING NUCLEIC ACID MOLECULES AND ANTI-HER3 ANTIBODIES

Applicants envision a wide variety of uses for the compositions of the present invention, including diagnostic and/or therapeutic uses of HER3, HER3 analogues and derivatives, HER3-encoding nucleic acid molecules and anti-HER3 antibodies.

HER3-encoding nucleic acid molecules or fragments thereof may be used as probes to detect and quantify mRNAs encoding HER3. Assays which utilize nucleic acid probes to detect sequences comprising all or part of a known gene sequence are well known in the art. HER3 mRNA levels may indicate emerging and/or exiting neoplasias as well as the onset and/or progression of other human diseases. Therefore, assays which can detect and quantify HER3 mRNA may provide a valuable diagnostic tool.

Anti-sense HER3 RNA molecules may be useful therapeutically to inhibit the translation of HER3-encoding mRNAs where the therapeutic objective involves a desire to eliminate the presence of a HER3. HER3 anti-sense RNA, for example, could be useful as a HER3 antagonizing agent in the treatment of diseases for which HER3 is a causative agent. Additionally, HER3 anti-sense RNAs may be useful in elucidating HER3 functional mechanisms.

HER3-encoding nucleic acid molecules may be used for the production of recombinant HER3 proteins and related molecules, as separately discussed in Section 5.1., *supra*.

## 5. 5. MOLECULAR BIOLOGY OF HER3

EGFR shares structural homology with HER2/*neu*; 83% amino acid identity in the cytoplasmic kinase domain, and 42% homology in the extracellular ligand binding domain where 49 of 50 cysteines are conserved. Applicants investigated whether the 78 amino acid form of Amphiregulin (AR) interacts with this putative receptor and homolog of EGFR, since the ligand for *neu* has not been identified. Experiments were performed on cells expressing high levels of human (SKBR-3 and BT474) or rat (B104-1-1 and DHFR-G8) *neu*. Direct binding, *in vitro* kinase assays, proliferation assays, *in vivo* phosphorylation, and receptor down-regulation assays all showed no association between the 78 amino acid form of AR and *neu* (G. Plowman, M. Shoyab, Y. Yarden, G. Todaro, and R. Weinberg, unpublished observations). It is possible that larger forms of AR, such as the 35 kD and transmembrane-bound forms of AR produced in cells transfected with the AR precursor gene, may interact with HER2/*neu* and/or HER3. Applicants are currently investigating this possiblity. For the present, however, the ligand for *neu* remains elusive.

Northern blot analysis on A431 and placental RNA provides evidence for the existence of other EGFR-related transcripts. A subclone of the human epidermoid carcinoma cell line A431 was isolated (A431-A3) after growth in soft agar. These cells have 30-fold increased sensitivity to AR inhibition compared with the parent line, yet had no alteration in the number or affinity of EGFR as measured by binding competition assays. Poly(A)$^+$ RNA was prepared from A431-A3 cells and a cDNA library constructed by specific priming with a 72-fold degenerate oligonucleotide complimentary to a stretch of 10 amino acids unique to the kinase domains of EGFR and *neu*. An upstream region of 7 amino acids, also conserved in EGFR and *neu*, was used to design a 96-fold degenerate oligonucleotide probe that was used to screen the cDNA library at low stringency conditions. Additional EGFR clones were eliminated by rehybridization at high stringency with a short EGFR cDNA probe spanning the same region of the kinase domain. Sixty-nine clones were partially sequenced, revealing three that encoded a closely related yet distinct member of the Human EGF Receptor (HER)/*neu* subfamily of receptor tyrosine kinases. A survey of several human tumor cell lines identified the breast carcinoma cell line, MDA-MB-361, to be a more abundant source of the 6 kb transcript than A431-A3 cells, so this line was used to isolate overlapping cDNA clones spanning the entire coding sequence of HER3. The HER3 transcript was also detected in a primary breast carcinoma, and normal colon, kidney, and brain.

Applicants' initial characterization of HER3 demonstrates the following conclusions: HER3 is a novel member of the EGFR subclass of receptor tyrosine kinases; HER3 does not interact with the ligands EGF and TGF-$\alpha$; prolonged exposure to EGF or AR reduces EGFR phosphorylation; and a 160 kD phosphoprotein is present in cells expressing both HER3 and membrane-associated AR, whereas the 170 kD EGFR is completely absent. Immunoprecipitation experiments suggest this 160 kD phosphoprotein is HER3 and that it is present only when coexpressed with the integral membrane form of the AR precursor.

Biochemical and biologic evidence suggests that members of the EGF/TGF-$\alpha$/AR family are not functionally equivalent. For example, TGF-$\alpha$ has an activity comparable to that of EGF in many *in vitro* assays: TGF-$\alpha$ has an EGFR binding affinity similar to that of EGF that results in autophosphorylation of EGFR, TGF-$\alpha$ induces similar changes in intracellular pH and calcium levels as EGF and it transduces a proliferative signal indistinguishable from that of EGF; however, TGF-$\alpha$ appears more potent than EGF in several *in vivo* assays, including the promotion of bone resorption, wound healing, and angiogenesis. Similarly, AR can be distinguished from EGF and TGF-$\alpha$ by its EGFR binding activity and by a number of biologic assays.

The identification of a novel member of the EGFR/*neu* subclass of RTKs reveals a greater repertoire by which the EGF/TGF-a/AR family of ligands can exert their diverse growth regulatory signals. Applicants' data suggests that some of the differences in biological activity between AR and EGF/TGF-$\alpha$ may result from AR's ability to interact differentially with both EGFR and HER3. Since EGFR and *neu* have been shown to act synergistically (Kobai et al., 1989, Cell 58: 287-92), it is also conceivable that HER3 may interact with either of these two receptors. Moreover, the disparity in response to these ligands may be reflected in the less conserved cytoplasmic region of HER3 compared with EGFR and *neu*. The consequences of the amino acid changes in the HER3 kinase region, as well as the lack of sequence homology to EGFR in its autophosphorylation domain, are under investigation. The carboxy-terminal region of EGFR appears to act as a competitive inhibitor for exogenous substrate phosphorylation and thereby serves to fine-tume its kinase activity (Margolis et al., 1989, J. Biol. Chem. 264: 10667-71; Honegger et al., 1988, EMBO 7: 3045-52). The more conserved homology between the cytoplasmic regions of EGFR and *neu*, as opposed to HER3, suggests these two receptors share a more similar function, whereas the distinctiveness of HER3 suggests that it may be subject to different regulation and might signal an alternate biochemical response.

## 6. EXAMPLE: cDNA CLONING OF HER3

## 6. 1. MATERIALS AND METHODS

## 6.1.1. OLIGONUCLEOTIDE PRIMERS/PROBES AND HYBRIDIZATION CONDITIONS

The oligonucleotide mixtures (including their degeneracy or length and corresponding amino acid residues) which were used for priming and screening the $\lambda$gt10 library were:

```
ARRD1        5'-ATCATCCARCAYTTDACCATDATCATRTA-3',   (72-fold,
             YMIMVKCWMI)

ARRD2        5'-GCCATCCAYTTDATNGGNAC-3',   (96-fold, VPIKWMA)

S729ERR      5'-TCGTCGACTCCTTCACCACTATCTCA-3',   (26-mer, EIVVKD)

S720ERR      5'-TGGCGTCGACCTATCTCAGCATCTCGGT-3',   (28-mer, DRDAEIV)

XSCT17       5'-GACTCGAGTCGACATCGATTTTTTTTTTTTTTTT-3',   (35-mer)

XSC          5'-GACTCGAGTCGACATCG-3',   (17-mer)
```

All oligonucleotides are designed to the anti-sense mRNA strand, except XSCT17 and XSC which are used as bidirectional primers. Degenerate residues are: D = A, G, or T; N = A, C, G, or T; R = A or G; Y = C or T. Oligonucleotides were labeled with $\gamma^{32}$P-ATP using T4 polynucleotide kinase ($3 \times 10^8$ cpm/$\mu$g), and hybridization was performed at 37°C, overnight with the probe at $2 \times 10^6$ cpm/ml in oligo hybridization mix (6XSSC, 5X Denhardt's, 0.15% sodium pyrophosphate, 0.1 mg/ml salmon sperm DNA, and 0.1 mg/ml tRNA). Multiple washes were done in 6XSSC, 42°C. cDNA probes were prepared by random prime $^{32}$P-labeling (specific activity of $5 - 25 \times 10^8$ cpm/$\mu$g), and hybridization was performed at 42°, overnight in southern hybridization buffer (50% formamide, 5X SSC, 25 mM NaHPO$_4$, 1x Denhardt's solution, 10% dextran sulfate and 20 $\mu$g/ml denatured herring sperm DNA) containing $2 \times 10^6$ cpm of $^{32}$P-labeled fragment per ml. Filters were washed extensively in 0.5x SSC, 0.1% SDS, 65°C, and autoradiographed overnight at -70°C.

## 6.1.3. cDNA LIBRARY CONSTRUCTION AND SCREENING

Total cellular RNA was extracted from A431 (subclone A3) and MD-MBA-361 cells and poly(A)$^+$ RNA was isolated. First strand cDNA synthesis was performed on A431-A3 RNA with reverse transcriptase using a 72-fold degenerate primer mixture based on the sequence YMIMVKCWMI (ARRD1, see underlined in FIG. 3). A cDNA library was constructed in λgt10 (B,C), and $3.0 \times 10^5$ recombinants were screened in duplicate on nitrocellulose filters. The filters were first probed at low stringency with $^{32}$P-labeled ARRD2, a 96-fold degenerate oligonucleotide (see underlined in Fig. 2). The filters were then stripped and reprobed at high stringency with $^{32}$P-labeled EGFR300, a cDNA fragment corresponding to amino acids 863 - 944 of the EGF receptor. Of 69 clones that differentially hybridized to ARRD2 and EGFR300, and were therefore chosen for sequence analysis, 3 were found to encode a novel EGFR-related protein (HER3), 6 encoded *neu*, and 10 spanned various regions of the EGF receptor. One HER3 clone was used as a probe to screen a λgt10 cDNA library derived from oligo(dT)-primed MD-MBA-361 RNA, and three additional cDNA clones were isolated, one with a 4.5 kb insert (pHER3-3b). This clone was estimated to lack approximately 500 bp of the 5' coding region, based on its high degree of homology with EGFR. To obtain the 5'-cDNA clone, we used polymerase chain reaction (PCR) as follows. Single stranded cDNA, synthesized from MD-MBA-361 RNA with S729ERR as a primer, was dA-tailed, and used as a template for PCR amplification using S720ERR, XSCT17, and XSC as primers. The PCR reaction products were cloned into pBluescript II SK+ (Stratagene), with the longest insert containing 701 bp of 5'-HER3 sequence (pBS5'HER3). All cDNA clones and several PCR generated clones were sequenced on both strands using T7 polymerase with oligonucleotide primers.

The PCR reaction products were cloned into pBluescript II SK+ (Stratagene), with the longest insert containing 701 bp of 5'-HER3 sequence (pBS5'HER3). All cDNA clones and several PCR generated clones were sequenced on both strands using T7 polymerase with oligonucleotide primers.

## 6.2. cDNA SEQUENCE OF HER3

The nucleotide sequence of HER3 contains an open reading frame of 1342 amino acids beginning with a consensus initiating methionine at nucleotide 199 (FIG. 1). The amino acids downstream of this methionine have the characteristics of a signal sequence, with the mature protein predicted to begin at S$^{20}$, followed by 1323 amino acids with a calculated Mr of 146,000. The 4026 nucleotide coding region is flanked by 198 nucleotides of 5' and 755 nucleotides of 3' untranslated sequences. No poly(A) tail is present, suggesting this is a partial cDNA sequence.

HER3 has all the structural features of a receptor tyrosine kinase with a single hydrophobic stretch of 32 amino acids characteristic of a transmembrane region that divides the sequence into a 612 residue extracellular ligand binding domain and a 677 amino acid carboxy-terminal cytoplasmic domain, and shows strong homology with other members of the tyrosine kinase family (FIG. 2, FIG. 3). The ligand binding domain can be further divided into 4 subdomains (I - IV), including two cysteine-rich regions (II, IV) that conserve all 48 cysteines present in the corresponding regions of the EGFR, and are thought to provide the structural backbone for domains I and III, which are implicated to define specificity for ligand binding. HER3 shares 40 - 50% identity with EGFR and 40 - 45% identity with *neu* in each of these subdomains. There are 10 potential *N*-linked glycosylation sites in the extracellular domain of HER3, conserving 5 of the 12 potential sites in EGFR and 3 of the 8 sites in *neu*. The cytoplasmic domain includes a stretch of basic residues flanking the membrane-spanning region, a consensus ATP binding site (G-X-G-X-X-G-$X_n$-K) and sequences homologous to other members of the tyrosine kinase family, followed by a hydrophilic carboxy-terminal tail containing 13 tyrosines (FIG. 3). The kinase domain of HER3 is most similar to EGFR and *neu* (60% and 62%) and shares lower homology (26 - 34%) with representatives of other classes of tyrosine kinases (FIG. 2). EGFR and *neu* have 83% amino acid sequence identity between their kinase domains, suggesting that, in this domain, they are more closely related to each other than they are to HER3.

The cytoplasmic domain of HER3 also has several unique features (FIG. 3). Protein kinase C-induced phosphorylation of EGFR occurs primarily on $T^{654}$, whereas $T^{669}$ has been implicated as a major site for phosphorylation of EGFR in response to EGF. Both of these residues are conserved in *neu*, but both are altered in HER3 ($A^{657}$ and $D^{672}$). Recent studies using EGFR mutants suggests that preventing phosphorylation at either of these residues has no effect on EGF binding, however, in certain cell lines, phosphorylation of EGFR $T^{654}$ by phorbol esters does appear to block EGF-induced mitogensis. HER3 already contains these "mutations" and might lack this negative control mechanism induced by protein kinase C.

Two additional amino acid changes are uniquely present in the HER3 kinase domain (FIG. 3). The catalytic domains of all known protein kinases contain certain residues that are either highly or completely conserved. HER3 contains most of these amino acids including; $G^{697}$, $G^{699}$, $V^{704}$, $K^{723}$, $N^{820}$, $DFG^{833-835}$, $E^{862}$, $D^{874}$, $G^{879}$, and $R^{938}$ however HER3 has nonconservative substitutions at $C^{721}$, $H^{740}$, and $N^{815}$. The latter two amino acids are present as glutamate and aspartate, respectively, in all known kinases; serine/threonine as well as tyrosine kinases (FIG. 2, FIG. 3). Strict conservation of these residues suggests they are important for catalytic activity, and the amino acid changes at these positions in HER3 predict it may have altered or absent kinase activity.

The carboxy-terminal 353 amino acids of HER3 contains 13 tyrosines, several of which are flanked by numerous charged residues. These features are characteristic of the autophosphorylation domains of EGFR and *neu*. This region of HER3 shares no significant primary sequence homology to either of these two family members and is approximately 30 - 50% longer. The sequence YEYM is repeated three times in this domain of HER3, possibly the result of gene duplication.

## 7. <u>EXAMPLE: TRANSIENT EXPRESSION OF HER3 IN COS CELLS</u>

### 7.1. <u>MATERIALS AND METHODS</u>

### 7.1.1. <u>CONSRUCTION OF EXPRESSION VECTORS AND TRANSFECTIONS</u>

Plasmid cHER3x was constructed for COS expression as follows. PCR clone pBS5'HER3 was partially digested with HincII and SmaI and religated to eliminate 157 bp of HER3 5'UTR. The 528 bp KpnI-SstI fragment was shuttled into pSPT18 (Boehringer) placing XbaI and SalI sites upstream of the coding sequence. This construct (pSP5'HER3c) contains 41 bp of HER3 5'UTR and the sequence encoding the first 174 amino acids of the HER3 precursor (to nucleotide 721). A 3' HER3 cDNA clone (pHER3-3b), was digested with EcoRI and KpnI, and the 3.7 kb fragment subcloned into pSPT18. The resultant construct (p3'HER3EK3.7) contains HER3 sequence from nucleotide 646 to the KpnI site (nucleotide 4287) 63 bp 3' of the stop codon. A 3.7 kb partial HindIII-XbaI (HindIII at nucleotide 662 in HER3 sequence) digested fragment was isolated from p3'HER3EK3.7 and ligated into pBluescript II SK+ (Stratagene) along with the SalI-HindIII fragment of pSP5'HER3c, generating pBSHER3 containing the complete HER3 coding sequence flanked by XbaI sites. The 4.1 kb XbaI fragment was inserted into pCDM8 (Invitrogen) placing the HER3 cDNA under the control of the cytomegalovirus promoter and enhancer (cHER3x). The expression construct pH3ARP (Plowman et al., 1989, submitted), contains the complete coding sequence for the amphiregulin precursor. The expression plasmids were grown in competent MC1061/P3 cells, and introduced into COS-1 cells using the DEAE-dextran method. Cell pellets were harvested 72-96 hr after transfection, and analyzed by immunoblotting as described (Plowman et al., 1989, submitted), except that samples were separated on 7% polyacrylamide gels. Polyclonal antisera was generated against synthetic peptides specific to the kinase domain of HER3 (PPDDKQLLYSEAKT, amino acids 841-854; GAEPYAGLRLAEVPD, amino acids 889-903).

EP 0 444 961 A1

## 7.1.2. IMMUNE COMPLEX KINASE ASSAYS

For immune complex kinase assays, 10 cm dishes of transfected COS cells (72 hours post-transfection) were washed three times in PBS, and scraped into 1 ml solubilization buffer (20 mM HEPES pH7.35, 150 mM NaCl, 10% glycerol, 1% Triton X-100, 1.5 mM MgCl$_2$, 1 mM EGTA, 2 mM phenylmethylsulfonyl fluoride, 1 µg/ml aprotinin). After 10 min incubation on ice, cell debris was removed by 12,000 x g centrifugation, 5 min, 4°C. The supernatant was then precleared with 50 µl of a 1:1 slurry of Staphylococcus aureus protein A-Sepharose suspended in solubilization buffer, and incubated with 5 µl EGFR1 monoclonal antibody (Amersham) and 50 µl S. aureus protein A-Sepharose, overnight, 4°C. Immune complexes were washed three times in HNTG (20 mM HEPES, pH 7.35, 150 mM NaCl, 0.1% Triton X-100, 10% glycerol), and stored at -70°C. For autophosphorylation, thawed immunoprecipitates were resuspended in 20 µl HNTG with 15 mM MnCl$_2$. Five µl 100 µM ATP with 15 µCi γ$^{32}$P-ATP was added and incubated for 1 min on ice. The reaction was terminated with 25 µl hot SDS sample buffer, and samples were analyzed on 7% SDS-polyacrylamide gels.

## 7.2. BIOCHEMICAL PROPERTIES OF RECOMBINANT HER3

To determine its biochemical properties, we inserted the complete coding sequence of HER3 into an expression vector under the control of the cytomegalovirus immediate-early promoter. This vector (cHER3x) was transfected into COS cells and the transiently expressed protein was detected by immunoblot analysis using antisera specific to the cytoplasmic domain of HER3. The recombinant protein migrated with an apparent Mr of 160,000, slightly less than the EGFR (FIG. 4). The HER3 expressing cells were tested for specific binding of $^{125}$I-EGF or $^{125}$I-TGF-α by cross-linking studies on whole cells. COS cells have high background levels of EGFR, however no differences were seen on binding to normal or HER3 expressing cells. The abundance of EGFR in COS cells, and difficulties obtaining high specific activity $^{125}$I-AR, complicated efforts to assess whether AR is a ligand for HER3. Instead a functional assay was used to demonstrate the specific effects of AR on this putative receptor.

Applicants have recently cloned the AR gene, and found the 78 amino acid secreted glycoprotein to be derived from a 252 amino acid transmembrane precursor by proteolytic cleavage. Further, the AR precursor is expressed in its membrane bound form in several human breast carcinoma cell lines and in COS cells transfected with an expression vector (pH3ARP) containing the complete AR coding sequence. This expression construct was used as a source of membrane bound AR to study its effect on the phosphorylation of EGFR and HER3. COS cells were transfected with either the HER3 or AR expression construct, or cotransfected with both. Two days after transfection, cells were put on serum free media for an additional 24 hr. The cells were solubilized and immunoprecipitated with a monoclonal antibody (Mab) to EGFR. Immune complexes were incubated with γ$^{32}$P-ATP and Mn$^{++}$, and resolved by 7% SDS-PAGE. COS cells, transfected with vector control or with HER3, showed strong phosphorylation of a single 170 kD protein (FIG. 4B, lanes 1-5), consistent with a high basal level of EGFR phosphorylation in COS cells. The amount of this phosphorylated protein was reduced significantly following prolonged treatment with EGF or AR, consistent with receptor internalization and degradation (Fig. 4B, lanes 2, 3, 6, 7). Unexpectedly, cells transfected with the AR precursor construct showed a complete loss of phosphorylated EGFR (Fig. 4B, lane 4) and absence of an $^{35}$S-labeled 170 kD EGFR. These cells exhibited an altered structural morphology, with extensive clumping into aggregates of interconnected islands, compared with the intact monolayer seen on transfection with the other constructs. Following prolonged exposure to EGF or AR, normal COS cells also displayed a disrupted monolayer, but with less aggregation than seen in cells expressing the AR precursor. Cotransfection of the HER3 and AR constructs resulted in a morphology similar to that seen on transfection with AR alone. Furthermore, the EGFR Mab was able to immunoprecipitate a new phosphorylated protein of MR 160,000 that comigrates with the recombinant HER3, and was only detected in COS cells expressing both AR and HER3 (Fig. 4B, lane 8).

These experiments suggested that EGFR shares an antigenic determinant with HER3, or that HER3 is coimmunoprecipitated with the EGFR Mab due to an association between these two RTK homologs. Alternatively, the 160 kD phosphoprotein could be a smaller form of EGFR, or a tightly associated substrate, that is specifically phosphorylated in the presence of both HER3 and the AR precursor, as seen with EGFR and phospholipase C-II (Margolis et al., 1989, Cell 57: 1101-07). To explore these possbilities further, in vivo phosphorylation experiments to label transfected COS cells and immunoprecipitated HER3 were performed with peptide antisera directed against its kinase domain. A 160 kD phosphoprotein was readily identified in COS cells transfected with HER3 and AR, but not in cells transfected with vector alone (FIG. 4C).

11

## Claims

1. A nucleotide sequence encoding a HER3 precursor comprising the nucleotide coding sequence substantially as depicted in FIG. 1 from about nucleotide number 1 to about nucleotide number 4978.

2. A nucleotide sequence encoding a HER3 precursor comprising the nucleotide coding sequence substantially as depicted in FIG. 1 from about nucleotide number 199 to about nucleotide number 4224.

3. A nucleotide sequence encoding a mature HER3 comprising the nucleotide coding sequence substantially as depicted in FIG. 1 from about nucleotide number 256 to about nucleotide number 4224.

4. A HER3 precursor comprising the amino acid sequence substantially as depicted in FIG. 1 from about amino acid residue number +1 to about amino acid residue number 1323.

5. A mature HER3 comprising the amino acid sequence substantially as depicted in FIG. 1 from about amino acid residue number +1 to about amino acid residue number 1323.

6. A method for producing HER3 comprising:
   (a) culturing a eucaryotic cell containing a nucleotide sequence encoding HER3 under the control of a second nucleotide that regulates gene expression so that a protein having HER3 biological activity is produced by the eucaryotic cell; and
   (b) recovering the HER3 from the culture.

7. The method according to claim 6 in which the nucleotide sequence encoding HER3 comprises the nucleotide sequence substantially as depicted in FIG. 1 from nucleotide number 1 to nucleotide number 4978.

8. The method according to claim 6 in which the nucleotide sequence encoding HER3 comprises the nucleotide sequence substantially as depicted in FIG. 1 from nucleotide number 199 to nucleotide number 4224.

9. The method according to claim 6 in which the nucleotide sequence encoding HER3 comprises the nucleotide sequence substantially as depicted in FIG. 1 from nucleotide number 256 to nucleotide number 4224.

## Fig. 1

Fig. 1 (con'd)

```
          960       R E S   P   I A P G   E P N G L T N K K L E E Y E L E P E L D L D L D
     Y L V I K R E S G P G I A P G P E P N G L T N K K L E E Y E L E P E L D L D L D
3121 TATCTGGTCATAAAGAGAGAGAGTGGGCCTGGAATAGCCCCTGGGCCAGAGCCCCATGGTCTGACAAACAAGAAGCTAGAGGAAGTAGAGCTGGAGCCAGAACTAGACCTAGACCTAGAC
          1000                  1010             1020                 1030
     L E A E E D N L A T I T L G S A L S L P Y S T L N R P R G S Q S L L S P S S G Y
3241 TTGGAAGCAGAGGAGGACAACCTGGCAACCACCACACTGGGCTCCGCCCTCAGCCTACCAGTTGGAACACTTAATCGGCCACGTGGGAGCCAGAGCCTTTTAAGTCCATCATCTGGATAC
          1040                  1050                 1060               1070
     M P M N Q G N L G G S C Q E S A Y S G S S S R C P R P Y S L M P M P R G C L A S
3351 ATGCCCATGAACCAGGGTAATCTTGGGGGGTCTTGCCAGGAGTCTGCAGTTTCTGGGAGCAGTGAACGGTGCCCCCGTCCAGTCTCTCTACACCCAATGCCACGGGGATGCCTGGCATCA
          1080                  1090                 1100                1110
     E S S E G H Y T G S E A E L Q E K V S M C R S R S R S R S P R P R G D S A Y H S
3481 GAGTCATCAGAGGGGCATGTAACAGGCTCTGAGGCTGAGCTCCAGGAGAAAGTGTCAATGTGTAGAAGCCGGAGCAGGAGCCCGGAGCCCACGGCCACGCGGAGATAGCGCCTACCATTCC
          1120  A      1130                 1140                1150
     Q R M S L L T P V T P L S P P G L E E E D Y N G T V N P D T K L K G T P S S R E
3601 CAGCGCCACAGTCTGCTGACTCCTGTTACCCCACTCTCCCCACCCGGGTTAGAGGAAGAGGATGTCAACGGTTATGTCATGCCAGATACACACCTCAAAGGTACTCCCTCCTCCCGGGAA
          1160   G        1170                1180                 1190
     G T L S S V G L S S V L G T E E E D E D E E Y E Y M N R R R R M S P P M P P R P
3721 GGCACCCTTTCTTCAGTGGGTCTCAGTTCTGTCCTGGGTACTGAAGAAGAAGATGAAGATGAGGAGTATGAATACATGAACCGGAGGAGGAAGGCACAGTCCACCTCATCCCCCTAGGCCA
          1200                 1210                 1220                1230
     S S L P E L L G Y E Y M D V G S D L S A S L G S T Q S C P L H P Y P I N P T A G T
3841 AGTTCCCTTGAGGAGCTGGGTTATGAGTACATGGATGTGGGGTCAGACCTCAGTGCCTCTCTGGGCAGCACACAGAGTTGCCCACTCCACCCTGTACCCATCATGCCCACTGCAGGCACA
          1240                 1250                 1260                1270
     T P D E D Y E Y M N R Q R D G G G P G G D Y A A M G A C P A S E Q G Y E E M R A
3951 ACTCCAGATGAAGACTATGAATATATGAATCGGCAACGAGATGGAGGTGGTCCTGGGGGTGATTATGCAGCCATGGGCGGCCTGCCCAGCTCTGAGCAAGGGTATGAAGAGATGAGAGCT
          1280                 1290                 1300                1310
     F Q G P G H Q A P H V H Y A R L K T L R S L E A T D S A F D N P D Y W H S R L F
4061 TTTCAGGGGCCTGGACATCAGGCCCCCCATGTCCATTATGCCCGCCTAAAAAACTCTACGTAGCTTAGAGGCTACAGACTCTGCCTTTGATAACCCTGATTACTGGCATAGCAGGCTTTTC
          1320
     P K A M A Q R T *
4201 CCCAAGGCTAATGCCCAGAGAACGTAACTCCTGCTCCCCTGTGGCACTCAGGGAGCATTTAATGGCAGCTAGTGCCTTTAGAGGGTACCGTCTTCTCCCTATTCCCTCTCTCTCCCAGGTC
4321 CCAGCCCCTTTTCCCCAGTCCCAGACAATTCCATTCAATCTTTGGAGGCTTTTAAACATTTTGACACAAAATTCTTATGGTATGTAGCCAGCTGTGCACTTTCTTCTCTTTCCCAACCCC
4441 AGGAAAGGTTTTCCTTATTTTGTGTGCTTTCCCAGTCCCAGACTCCTCAGCTTCTTCACAGGCACTCCTGGAGATATGAAGGATTACTCTCCATATCCCTTCCTCTCAGGCTCTTGACTACT
4561 TGGAACTAGGCTCTTATGTGTGCCTTTGTTTCCCATCAGACTGTCAAGAAGAGGAAAGGGAGGAAAACCTAGCAGAGGAAAGTGTAATTTTGGTTTATGACTCTTAACCCCCTAGAAAGAC
4681 AGAAGCTTAAAATCTGTGAAGAAAGAGGTTAGGAGTAGATATTGATTACTATCATAATTCAGCACTTAACTATGAGCCAGGCATCATACTAAACTTCACCTACATTATCTCACTTAGTCC
4801 TTTATCATCCTTAAAACAATTCTGTGACATACATATTATCTCATTTTACACAAAGGGGAAGTCGGGCATGGTGGCTCATGCCTGTAATCTCAGCACTTTGGGAGGCTGAGGCAGAAGGATT
4921 ACCTGAGGCAAGGAGTTTGAGACCAGCTTAGCCAACATAGTAAGACCCCCATCTCTTT
```

14

# Fig. 2

# Fig. 3

Fig. 4

# Fig. 1

European Patent
Office

## EUROPEAN SEARCH REPORT

Application Number

| | DOCUMENTS CONSIDERED TO BE RELEVANT | | EP 91301737.2 |
|---|---|---|---|

| Category | Citation of document with indication, where appropriate, of relevant passages | Relevant to claim | CLASSIFICATION OF THE APPLICATION (Int. Cl.5) |
|---|---|---|---|
| P,X | PROCEEDINGS OF THE NATIONAL ACADEMY OF SCIENCES OF THE UNITED STATES OF AMERICA, vol. 87, No. 13, July 1990 (Baltimore, USA) G.D. PLOWMAN et al. "Molecular cloning and expression of an additional epidermal growth factor receptor-related gene" pages 4905-4909 * Totality * | 1-9 | C 12 N 15/12 C 07 H 21/04 C 12 P 21/00 |
| P,A | WO - A1 - 90/14 357 (GENENTECH, INC.) * Abstract * | 1 | |
| D,A | GB - A - 2 214 185 (ONOCOGEN) * Abstract * | 1 | |
| D,A | MOLECULAR AND CELLULAR BIOLOGY, vol. 6, No. 5, May 1986 (Washington DC) D.F. STERN et al. "p185, a Product of a new Proto-Oncogene, Is a Receptorlike Protein Associated with Tyrosine Kinase Activity" pages 1729-1740 * Totality * | 1 | TECHNICAL FIELDS SEARCHED (Int. Cl.5) C 12 N C 07 H C 12 P |
| D,A | SCIENCE, vol. 232, No. 4758, June 27, 1986 (Washington DC) T. AKIYAMA et al. "The Product of the Human c-erbB-2 Gene : A 185-Kilodalten Glycoprotein with Tyrosine Kinase Activity" | 1 | |

The present search report has been drawn up for all claims

| Place of search | Date of completion of the search | Examiner |
|---|---|---|
| VIENNA | 10-06-1991 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**EUROPEAN SEARCH REPORT.**

| | DOCUMENTS CONSIDERED TO BE RELEVANT | | -2-<br>EP 91301737.2 |
|---|---|---|---|
| Category | Citation of document with indication, where appropriate,<br>of relevant passages | Relevant<br>to claim | CLASSIFICATION OF THE<br>APPLICATION (Int. CL5) |
| A | pages 1644-1646<br>* Totality *<br>--<br>DE - A1 - 3 910 084<br>(ONCOGEN LTD.)<br>* Abstract *<br>-- | 1 | |
| D,A | ANNUAL REVIEW OF BIOCHEMIS-<br>TRY, vol. 54, 1985 (Washing-<br>ton DC)<br>T. HUNTER et al. "Protein-<br>tyrosine kinases"<br>pages 897-930<br>* Totality * | | |
| | | | TECHNICAL FIELDS<br>SEARCHED (Int. CL5) |

The present search report has been drawn up for all claims

| Place of search | Date of completion of the search | Examiner |
|---|---|---|
| VIENNA | 10-06-1991 | WOLF |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)